# EUROPEAN PATENT APPLICATION

(11) **EP 4 497 448 A1**
(43) Date of publication of application: **29.01.2025**
(21) Application number: 23315300.6
(22) Date of filing: 28.07.2023
(51) Int. Cl.: A61L 15/58

(54) **ADHESIVE-COMPRISING LAYER**

(71) Applicant: Advanced Silicone Coating, 69330 Pusignan (FR)
(72) Inventor: Lecoeuvre, Jean-François, 42290 SORBIERS (FR); Schmidt, Christian, 73466 LAUCHHEIM (DE)
(74) Representative: Littolff, Denis

(57) **Abstract**

The invention relates to a new adhesive-comprising layer showing excellent properties when being adhered to a surface such as the skin. The layer can for example be used for the preparation of articles such as wound dressings which are used for promoting/supporting the process of the healing of a wound.

## Description

The invention relates to a new adhesive-comprising layer showing excellent properties when being adhered to a surface such as the skin. The layer can for example be used for the preparation of articles such as wound dressings which are used for promoting/supporting the process of the healing of a wound.

A wound can be regarded as the separation of the contiguity of skin cells, wherein this may come along with a loss of tissue substance.

The healing of wounds is based on the ability of the skin to regenerate tissue such as epithelial tissue, connective and supporting tissue. The regeneration of tissue is a complex occurrence of cell activities overlapping with each other, wherein said cell activities promote the healing process step by step. A suitable wound dressing may help to provide a favorable environment for said healing process.

Such wound dressings have to be applied on a surface such as a wound or the skin surrounding said wound, which can be also considered as wound margins. On the one hand, it is essential that the wound dressing adheres well to said surface to prevent an undesired slipping or even removal of the wound dressing. On the other hand, it is also important to ensure that, once a wound dressing has to be removed, for example to change the dressing, the removal of the dressing does not negatively affect the surface of the skin or the wound. In particular, the peeling off of the wound dressing in line with a change thereof should not cause pain and/or even damage to the wound itself and/or its borders. In fact, the properties of the layer facing the wound appear to be essential to ensure the above-indicated adherence properties of a wound dressing.

The most simple and basic versions of wound dressings provide just a wound pad and an adhesive backing. While the backing stretches beyond the borders of the pad and allows adherence to healthy skin surrounding the wound, the pad simply has the function of absorbing liquids from the wound. Such dressings are offered for a low price and provide basic care for minor injuries. However, this approach comes with drawbacks. First of all, an untreated pad may stick to the wound and tear the wound open during change of the dressing (traumatic removal). Second, because the pad is not treated with an adhesive, the overall adhesive strength of the dressing does not reach its optimal value. Furthermore, such a pad is detrimental in case a dressing is treated with active ingredients (like compounds which are antimicrobial or promote healing) as the pad may lack direct contact to the wound and the ingredients are not released.

Within the field of wound dressings, polysiloxanes (herein also referred to as (poly)silicones) are known to have several advantages such as their adhesive properties. In addition, they exhibit atraumatic properties and provide a pleasant skin feeling. Further, the properties of such silicones can depend on their structures. EP 2 350 220 B2 relates to a method for making crosslinked silicone-based material by mixing the ingredients in an extruder to from a hot-melt coated composition and subsequently subjecting the composition to electron beam radiation, and to silicone materials obtained by said method. Emilie Gautriaud et al.: "Effects of Sterilization on the mechanical properties of silicone rubbers", Saint-Gobain, Northborough R&D Center MA, USA, Saint-Gobain Performance Plastics, 2009, www.biopharm.saint-gobain.com, pages 1 to 7 *inter alia* describe the influence of radiation on silicone. As can be seen from the scheme below, irradiating silicones in line with sterilization of the product can lead to different kinds of crosslinking. However, such a product seems to be improvable with regard to the peeling properties, such as the peel strength and peeling condition, once peeling (removal from skin or wound) is desired.

Effect of gamma and electron radiations on silicone polymer chains. 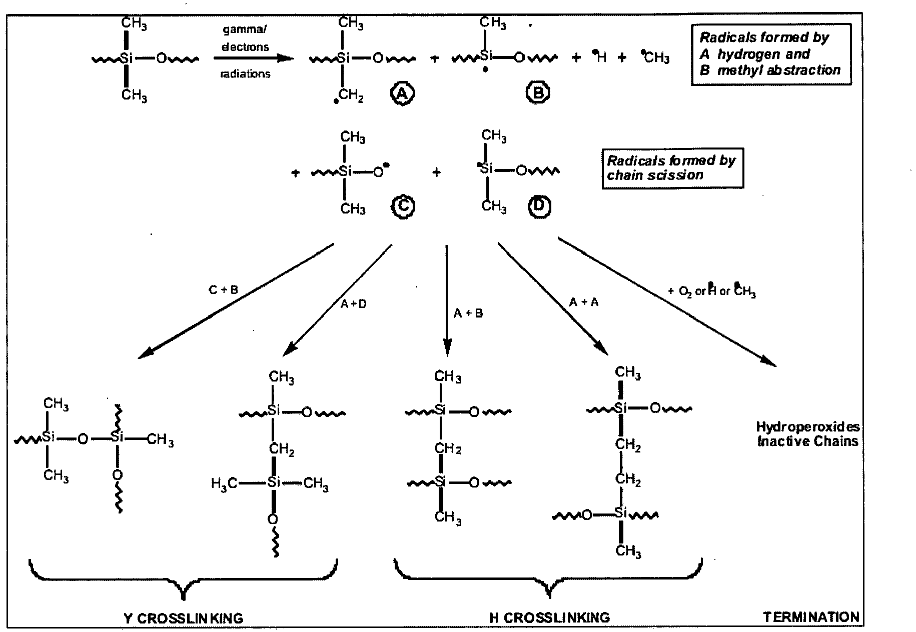 The wound dressing Bitain^{®} Silicone from Coloplast AB is a sandwich designed dressing that comprises a perforated silicone wound contact layer with differential adhesiveness in the skin area surrounding the wound and the wound area, which is achieved by different perforation patterns in said areas. In particular, on the one hand said dressing comprises small holes and a low open area percentage in the skin area surrounding the wound, which leads to a high adhesion capacity or peel strength in said area. On the other hand, the center of the dressing has bigger holes and a high open area percentage, which leads to a high absorption capacity in the wound area, but to a decreased adhesion capacity or peel strength. In any case, such a wound contact layer seems to be improvable, since for achieving a lower adhesiveness in the wound area there is a need of increasing the open area percentage which in turn increases the risk of new formed cell attachment through the wound contact layer to the overlying layer such as a wound pad layer (reduction of atraumatic properties). Moreover, the preparation of such a wound contact layer requires complex perforation tools which further have to be changed depending on the kind of holes to be punched and/or dressing dimensions and shape. This increases the burden during the production.

Thus, there is still a need for layer comprising an adhesive material which can, for example, be used for the manufacture of a wound dressing which, once applied to the skin/wound margin and/or wound/wound bed, remain there until its secure removal and/or additionally prevents cell attachment. Moreover, such a layer should be easily manufacturable in large scale and/or without the need of complex equipment.

Hence, it was an object of the present invention to overcome the above drawbacks.

In particular, it was an object of the present invention to provide a layer which can for example be used in a wound dressing which is capable of ensuring a peel strength, in particular in the area attached to skin surrounding the wound (periphery of the layer), that ensures continued adhesion at the place the wound dressing has been applied to.

Further, a layer that substantially prevents any cell attachment to the layer itself or even to an overlying layer should be provided. In other words, it is an object of the present invention to provide a layer with a structure that prevents the wound dressing for example from slipping or even removal at an undesired time.

Further, a layer with a structure should be provided which ensures a safe removal from the wound bed without negatively affecting the surface of the wound to which it has been applied.

### Summary

The present invention has unexpectedly solved the above objectives by a new layer comprising a medically acceptable adhesive material and having areas with different peel strengths of the medically acceptable adhesive material.

Thus, the subject of the present invention is a layer comprising one medically acceptable adhesive material on the same surface, wherein the peel strength of the medically acceptable adhesive material in one area of the layer is lower than the peel strength of the medically acceptable adhesive material in another area of the layer.

Another aspect of the invention is a method for preparing a layer according to the invention comprising the steps of
(I) providing a layer comprising one medically acceptable adhesive material
(II) applying an energy dose on at least one area of the medically acceptable adhesive material to reduce the peel strength of the medically acceptable adhesive material in this area by irradiating with an electron beam.

Another aspect of the invention is a laminate comprising (a) the layer according to the present invention and (b) a substrate, wherein layer (a) is located on one side of the substrate (b).

Another aspect of the invention is an assembly of the laminate according to the present invention and a textile or a polymer-based foil.

Finally, the invention is directed to the use of the layer according to the invention, the laminate according to the invention or the assembly according to the invention as (A) a wound contact layer of a wound dressing, preferably in an absorbent wound dressing, wherein the adhesive layer is preferably configured to comprise a wound contact area and a skin contact area and/or (B) a backing layer of a wound dressing, preferably as a backing layer of an island-like wound dressing.

### Detailed Description of the Invention

The present layer comprises a medically acceptable adhesive material. An adhesive material can be regarded as a substance which, when applied to one or both surfaces of two distinct subjects, binds them together and prevents their unintended separation. The adhesive material comprised in or on the layer is medically acceptable. A medically acceptable adhesive material is an adhesive material which substantially does not negatively affect the environment/surface of the skin/body to which it may be applied. Thus, when for example applied to a wound, the medically acceptable adhesive material does not release inadvertently or grow together with the wound tissue, since both can be detrimental to wound healing. Also, a medically acceptable adhesive material suitable for contacting a wound is biocompatible and nontoxic.

Further, the peel strength of the medically acceptable adhesive material is lower in one area than in another area of the layer. Preferably, the peel strength of the medically acceptable adhesive material in the center of the layer (area corresponding to the wound bed while in use) is lower than the one in the periphery of the layer (area corresponding to the skin surrounding the wound while in use). By having a lower peel strength of the medically acceptable adhesive material of the layer in center of the layer (area corresponding to the wound bed while in use), a traumatic adhesion of the layer to the wound bed can be advantageously prevented or even avoided. On the other side, a higher peel strength of the medically acceptable adhesive material of the layer in the periphery of the layer (area corresponding to the skin surrounding the wound while in use) ensures continuance at the place the layer has been applied. to.

It is preferred that the value of the peel strength of the medically acceptable adhesive material in the center of the layer (area corresponding to the wound bed while in use) is reduced by 25%, preferably by 50%, more preferably by 75% of the value of the peel strength of the medically acceptable adhesive material in the periphery of the layer (area corresponding to the skin surrounding the wound while in use). In other words, the value of the peel strength of the medically acceptable adhesive material in the center of the layer (area corresponding to the wound bed while in use) is 75%, preferably 50%, more preferably 25% of the value of the peel strength of the medically acceptable adhesive material in the periphery of the layer (area corresponding to the skin surrounding the wound while in use). The peel strength is determined as explained in the experimental section below.

In context of the present invention, the lower peel strength which is suitable for atraumatic wound contact, may be in a range of 0,1 to 1,25 N / 25 mm, preferably 0,15 to 1N / 25 mm, more preferably 0,15 to 0,8 N / 25 mm and most preferably 0,2 to 0,6 N / 25 mm. The higher peel strength which is suitable for adhering the layer to healthy skin surrounding a wound, may be in a range of 1,3 to 4 N / 25 mm, preferably 1,4 to 3,5 N / 25 mm, more preferably 1,6 to 3 N / 25 mm and most preferably 1,7 to 2,2 N / 25 mm. An example of suitable combination of peel strength is 0,1 to 0,3 N / 25 mm for the lower value and 1,6 to 2,2 N / 25 mm for the higher value.

In a preferred embodiment, the layer is a wound contact layer. This means that the layer is directly applied over the wound bed and/or the skin surrounding the wound. In context of this invention a side or surface facing during use towards the wound may be called proximal while the opposite side or surface (facing away from the wound during use) may be called distal.

The medically acceptable adhesive material comprised in the layer can be any medically acceptable adhesive known to the skilled person. Examples of medically acceptable adhesive materials are acrylate-based adhesives, epoxide-based adhesives, urethane-based adhesives, and silicone-based adhesives. It is preferred that the medically acceptable adhesive material comprised in the layer is an acrylate-based adhesive or a silicone-based adhesive material or a combination of both. More preferred, the medically acceptable adhesive comprised in the layer is a silicon-based adhesive material. Such a silicon-based adhesive material may for example be a sticky silicon gel, preferably comprising polydimethylsiloxane. Preferably the silicon-based adhesive material allows the formation of cross-linking bonds within the silicon-based adhesive.

In a more preferred embodiment of the invention, the layer comprising a medically acceptable adhesive material is made of or covered with the medically acceptable adhesive material, preferably the silicon-based adhesive material. In an even more preferred embodiment of the invention, the layer comprising a medically acceptable adhesive material is made of the medically acceptable adhesive material, preferably the silicon-based adhesive material.

In a preferred embodiment, the layer comprising a medically acceptable adhesive material has a thickness of from 20 µm to 225 µm, more preferably from 30 µm to 215 µm, in particular from 40 µm to 200 µm.

Preferably, the layer has a thickness of about 45 µm, about 50 µm, about 60 µm, about 70 µm, about 80 µm, about 90 µm, about 100 µm, about 110 µm, about 120 µm, about 130µm, about 140 µm, about 150µm, about 160µm, about 170µm, about 180 µm or about 190 µm.

In a further preferred embodiment, the layer is made of a medically acceptable silicone-based adhesive and has a thickness of 20 µm to 225 µm, more preferably from 40 µm to 200 µm.

In a preferred embodiment, the layer according to the invention has perforation holes, wherein the perforation holes are evenly distributed over the whole area of the layer. A perforation hole can be considered as an opening going through the layer. Evenly distributed over the whole area of the layer means that every part of the area has the same amount (in number and area) of openings. Thus, there is no part of the area which has more and/or bigger openings than another part. Preferably, the distance between all evenly distributed openings is constant or substantially the same, thereby creating a pattern of evenly distributed openings. The distance may be for example 0.1 to 5 mm, more preferably 0,3 to 3 mm and most preferably 0,4 to 2 mm.

Perforation holes can be made by any device suitable for the perforation of a layer such as nails, needles, laser, or a punching device. Alternatively, perforation can be carried out by die cutting, kiss cutting and by ultrasonic cutting.

The opening of a perforation hole can be any form. In other words, the form of the opening of a perforation hole can for example be rectangular, square, circular, elliptic, triangular, pentagonal, hexagonal, octagonal, or diamond-shaped. In a preferred embodiment the openings of the perforation holes are square, circular, or elliptic, preferably circular.

The openings of the perforation holes have a size of between 1.75 mm² and 12.5 mm², preferably between 2.0 mm² and 8.5 mm², in particular between 2.25 mm² and 5.0 mm².

In case that the perforation hole is circular, the opening size of a perforation hole between 1.75 mm² and 12.5 mm² substantially corresponds to a diameter of the opening hole of about 1.5 mm to about 3.9 mm. Correspondingly, again if circular, the opening size of a perforation hole between 2.25 mm² and 5.0 mm² substantially corresponds to a diameter of the opening hole of about 1.7 mm to about 2.5 mm. By the above diameter of the perforation holes, it can be ensured that the peel strength in the area of the periphery of the layer (area corresponding to the skin surrounding the wound while in use) is high enough that the wound dressing adheres well to said surface. In addition, a perforation with the above size holes enables a good moisture vapor transmission rate such that the wound can be kept moist to improve the healing process and that the exudate can be removed from the wound itself, wherein it is additionally ensured that the formation of new formed cell attachment through the layer to optional the overlying layers such as a wound pad layer is significantly reduced or even prevented.

In a preferred embodiment of the invention, the perforated surface is between 10% and 50% of the total surface of the layer. In other words, the sum of the areas of the opening of the perforation holes is between 10% and 50%, preferably between 12% and 45%, more preferably between 15% and 30% of the total surface of the layer. These areas can also be referred to as open (or opened) areas of the layer due to the absence of any material in this area.

The sum of the areas of the opening of the perforated holes is preferably about 15%, about 16%, about 17%, about 18%, about 19%, about 20%, about 21%, about 22%, about 23%, about 24%, about 25%, about 26%, about 27%, about 28%, about 29% or about 30%, more preferably about 25% of the total surface of the layer.

The invention is further directed to a method for preparing a layer according to the invention comprising the steps of
(I) providing a layer comprising one medically acceptable adhesive material
(II) applying an energy dose on at least one area of the medically acceptable adhesive material to reduce the peel strength of the medically acceptable adhesive material in this area by irradiating with an electron beam.

In step (I) a layer comprising one medically acceptable adhesive material is provided. As far as the layer comprising one medically acceptable adhesive material is concerned, the same as described above applies. Thus, in an embodiment, the layer comprising one medically acceptable adhesive material used in step (I) can, for example, be a layer made of silicone-based adhesive. Further, step (I) may optionally comprise a further step (Ii) of perforating the layer comprising one medically acceptable adhesive material. The perforating of the layer in said step (Ii) can for example be carried with by die cutting, kiss cutting and by ultrasonic cutting, preferably kiss cutting.

In step (II) an energy dose is applied on at least one area of the medically acceptable adhesive material to reduce the peel strength of the medically acceptable adhesive material in this area by irradiating with an electron beam.

Preferably, the area on which the energy dose is applied is not the entire surface on which the medically acceptable adhesive is located. The term "entire surface on which the medically acceptable adhesive is located" may mean only one of two sides of the layer, preferably the side which is intended to face towards the wound during use. For example, the area on which the energy dose is applied may be 10 to 30%, or 10 to 40%, or 10 to 50% or 10 to 60% or 10 to 70% of the entire surface of the layer during production (for example in relation to the bulk material before cutting the product to its final dimensions) or of the layer in the final product (for example in a wound dressing).

According to an embodiment of the above given method during step (II) a first energy dose is applied on a first area of the medically acceptable adhesive and a second energy dose is applied on a second area of the medically acceptable adhesive, wherein the second energy dose is higher than the first energy dose resulting in a higher peel force strength in the first area than in the second area. In a preferred embodiment, in step (II) just the area of the layer of the medically acceptable adhesive material supposed to have a lower peel strength is irradiated with an electron beam (e-beam). In a preferred embodiment, the area of the center of the layer (area corresponding to the wound bed while in use) is irradiated with an electron beam (e-beam) having a higher energy dose than the electron beam (e-beam) irradiating the area of the periphery of the layer (area corresponding to the skin surrounding the wound while in use). Further, similar as described above step (II) may be followed by an optional step (IIi) of perforating the layer comprising one medically acceptable adhesive material after irradiation. The perforating of the layer in said step (IIi) can for example be carried with by die cutting, kiss cutting or by ultrasonic cutting, preferably kiss cutting.

An electron beam as used in step (II) can be generated by any conventional means known in the art including cathode-ray tubes and electron beam processing systems (EPS). Examples are an e-beam system from Wasik Associates, devices offered by the NHV Corporation like the EBC-250-20-35, the Curetron-150 and the EB-XW, devices offered by the Electron Crosslinking AB like the EC-LAB 400 or the EB 150 50 240x100 as well as the Rhodotron from IBA or radiation produced at the ferrix^{®} facility.

The term "energy" is to be understood as the amount of energy emitted by an e-beam device and given in eV. The emitted energy may be in the range of 100 keV to 10 MeV. Preferably the amount of emitted energy is in the range of 100 keV to 500 keV as the devices required for this range are smaller and cheaper than devices which are able to produce electron beams with an energy of more than 500 keV.

The term "energy dose" is to be understood as the amount of energy absorbed by a material through irradiation with an e-beam device and given in Gy. In context of this invention, the term "energy dose" is more decisive than the term energy as it is the "energy dose" which determines the peel strength of the adhesive.

In a preferred embodiment, the energy dose in step (II) is 3 to 75 kGy, preferably 5 to 75 kGy, more preferably 8 to 60 kGy, most preferably the energy dose is 3 to 50 kGy. The energy dose may be about 3 kGy, about 5 kGy, about 10 kGy, about 15 kGy, about 20 kGy, about 25 kGy, about 30kGy, about 35 kGy, about 40 kGy, about 45 kGy, about 50 kGy, about 55 kGy, about 60 kGy, about 65 kGy, or about 70 kGy, more preferably about 25 kGy, 40 kGy or 60 kGy. Preferably, the peel force strength of the medically acceptable adhesive material is reduced by 1 to 5% by application of 1 to 3 kGy or is reduced by 10 to 15% by application of 5 to 10 kGy, or is reduced by 40 to 50% by application of 12 to 15 kGy or is reduced by 80 to 90% by application of 20 to 30 kGy or is reduced by 90 to 99% by application of 45 to 60 kGy thereby adjusting the peel force to the desired purpose.

In case a first energy dose is to be applied on a first area and a second energy dose is to be applied on a second area, the first energy dose may for example be 3 to 15 kGy and the second energy dose may for example be 30 to 75 kGy.

In an embodiment, during applying the energy dose respectively during the irradiation in step (II), the adhesive comprised by the layer may be covered by a liner. This offers the advantage of improved manageability of the layer (or the laminate as described below) during a high throughput process like for example on an assembly line. If both surfaces of the layer (or laminate) are covered with an adhesive, liners may be applied on each side. The material used for the liner should be as far as possible permeable towards the irradiation or alternatively the amount of applied energy should be increased accordingly. It is recommended to measure a potential absorption by the liner upfront and to increase the amount of applied energy accordingly if needed. For further processing like integration of the layer into a laminate (and/or laminate) into a wound care product the liner may be removed or - after optional perforation - replaced. A liner covering the adhesive only during a production process for achieving a final product may be called protective liner and may be made for example out of (optionally coated) paper. A liner covering the adhesive in the final product may be called release liner and foils like for example PET foils may be used as release liners.

In a further preferred embodiment, just the area of the layer of the medically acceptable adhesive material supposed to have the lower peel strength is irradiated, while the area of the layer supposed to have the higher peel strength is not irradiated. Such a structure can for example be achieved by focusing the electron beam on the area of the layer of the medically acceptable adhesive material supposed to have the lower peel strength and/or protecting the layer area supposed to have the higher peel strength from the electron beam for example by a corresponding mask.

Generally, in context of the present invention masks can be used to either prevent or reduce the irradiation of an area of the layer. Therefore, masks are suitable means to generate areas on the layer which have a higher peel strength than areas which are not shielded by a mask towards irradiation. Preferably a suitable mask will comprise or consist of one or more metals or one or more alloys. Metals are suitable because they are decent conductors of electricity and heat and thereby a sufficient distribution of the absorbed energy is possible. Preferably, the mask comprises or consists of a heat resistive high atomic number metal like for example hafnium, tantalum, lead or tungsten. These metals are able to absorb high energy doses. In case lower energy doses are to be applied (e.g. 5 to 50 kGy) masks comprising or consisting of aluminium, titan, vanadium, chrome, iron, nickel, copper, zinc or stainless steel deliver adequate results. A mask may have a thickness from 0.5 mm to 6 cm, preferably 1 mm to 5 cm, more preferably 3 mm to 4 cm and most preferably 5 mm to 3 cm. The surface of the mask usually will depend on the surface of the layer to be irradiated. The surface of the mask may for example be 25 cm² to 10.000 cm², preferably 50 cm² to 5.000 cm², more preferably 100 cm² to 2500 cm².

Consequently, the method for preparing a layer according to the invention may involve the use of a mask during step (II) wherein no energy or a lower energy dose is delivered to the respective area of the layer with the use of a mask positioned between the irradiation device and the layer such that a part or all of the emitted energy is absorbed by the mask. In this context, the mask offers the advantage that the applied irradiation may have a constant energy level and the produced adhesive layer still possesses at least two areas of different peel strength. The production process using a mask may be an assembly line process and / or a high throughput process wherein the irradiation device and the mask stay at a fixed position and the layer (or layers) are moved on an assembly line or are fed into a treatment chamber.

It is possible to use one, two, three or more masks at the same time or consecutively during the irradiation. Each used mask may have one to hundred holes, preferably one to fifty holes, more preferably four to twenty holes. The holes allow the passage of the e-beam irradiation and may have different shapes including stripes, circles, ellipses and rectangles.

Another aspect of the invention is a laminate comprising (a) the layer according to the present invention and (b) a substrate, wherein layer (a) is located on one side of the substrate (b). The laminate may be a so-called cold laminate, which is bonded by adhesive forces. Cold laminates offer the advantage that (in contrast to thermally bonded laminates) due to the absence of weld seams the surface is completely even wherein weld seams may be irritant when contacting the skin.

As far as the layer relating to (a) is concerned, it applies the same as described above with regard to the layer according to the invention.

Further, the present laminate comprises a substrate (b). Preferably, the substrate is a flat substrate having two surfaces. In line with the present application a substrate can be considered as a carrier comprising an inert material. Inert materials suitable for a substrate are known in the art. Non-limiting examples of suitable inert materials are polymers, such as polyethylene-based materials, polypropylene-based materials, polyester-based materials, such as materials from polyethylene terephthalate (PET), polyamide-based materials, polyvinylchloride-based materials, polyacrylate-based materials, polymethacrylate-based materials, polyurethane-based materials, such as materials from polyester urethane or polyether urethane, silicone-based materials and mixtures thereof. In a preferred embodiment the substrate comprises a polyurethane-based material. More preferably the substrate is a polyurethane-based membrane. Most preferably the membrane is made of thermoplastic polyurethane (TPU). The TPU based membrane may be derived from blow extrusion. If the laminate is perforated than the membrane is also perforated. Preferably the perforations of the laminate and the membrane are coextensive. The membrane may be permeable for water and / or water vapor but in case of a perforated laminate the membrane may also be impermeable for water and / or water vapor which facilitates the production of the laminate.

Further, the substrate (b) can have any form as long as the layer (a) can be located/coated on one side of the substrate. Preferably the substrate is a film.

In a preferred embodiment the substrate (b) has a thickness of 5 µm to 50 µm, more preferably 10 µm to 40 µm, in particular 20 µm to 30 µm.

Preferably the substrate (b) has a thickness of about 21 µm, about 22 µm, about 23 µm, about 24 µm, about 25 µm, about 26 µm, about 27 µm, about 28 µm or about 29 µm, especially about 25 µm.

In a further preferred embodiment, the substrate comprises polyurethane-based material, preferably in form of a film or membrane, and has a thickness of 5 µm to 50 µm, more preferably from 10 µm to 40 µm.

When the layer (a) has perforation holes, the substrate (b) has also preferably perforation holes. In that case, the layer (a) and the substrate (b) preferably have the same pattern of perforation as far as location and size of the holes are concerned. In other words, the laminate is considered to be perforated by the perforation holes. Perforation of the laminate can be carried out with the same devices as described above with regard to the perforation of the layer (a).

Layer (a) is located on one side of substrate (b). In a preferred embodiment, the laminate is construed such that the layer (a) is the wound contact layer. In other words, one surface of layer (a) is arranged such that it is facing towards the wound during use.

In an embodiment, the laminate can comprise a second layer (c) comprising a medically acceptable adhesive material. As far as the medically acceptable adhesive material comprised in second layer (c) is concerned, generally the same applies as described above with regard to the medically acceptable adhesive material comprised in layer (a). The adhesive materials within the laminate may be of the same kind of adhesive or two different adhesives like for example acrylate-based adhesive and silicone-based adhesive. The adhesive materials may be covered with removable liners whenever this facilitates the handling or processing of the laminate.

Thus, the medically acceptable adhesive materials comprised by second layer (c) can for example be acrylate-based adhesive material, epoxide-based adhesive material, urethane-based adhesive material or silicone-based adhesive material.

The medically acceptable adhesive material comprised by second layer (c) can preferably be the same adhesive as the medically acceptable adhesive material comprised by layer (a).

Alternatively preferred, the medically acceptable adhesive material comprised by second layer (c) can be another adhesive as the medically acceptable adhesive material comprised by layer (a). It is preferred that the medically acceptable adhesive material comprised in the second layer (c) is an acrylate-based adhesive material or a silicone-based adhesive material. More preferred, the medically acceptable adhesive material comprised in second layer (c) is an acrylate-based adhesive material.

In a preferred embodiment the second layer (c) consists of a medically acceptable adhesive material. In a more preferred embodiment, the second layer (c) comprises or consists of an acrylate-based adhesive material, the substrate (b) comprises or consists of a polyurethane-based material and the layer (a) comprises or consists of a silicone-based adhesive material.

In a preferred embodiment, second layer (c) has a thickness of 5 µm to 100 µm, more preferably 10 µm to 70 µm, in particular 20 µm to 40 µm, especially about 30 µm.

Preferably the second layer (c) has a thickness of about 21 µm, about 22 µm, about 23 µm, about 24 µm, about 25 µm, about 26 µm, about 27 µm, about 28 µm, about 29 µm, about 30 µm, about 31 µm, about 32 µm, about 33 µm, about 34 µm, about 35 µm, about 36 µm, about 37 µm, about 38 µm or about 39 µm.

In a further preferred embodiment, second layer (c) comprises a medically acceptable acrylate-based adhesive and has a thickness of 5 µm to 100 µm, in particular 20 µm to 40 µm.

In a preferred embodiment, layer (a) is located on one side of the substrate (b) and second layer (c) is located on the opposite side of the substrate (b). In a preferred embodiment, second layer (c) is located onto the opposite side of the substrate (b) onto which layer (a) is applied. Thus, second layer (c) is optionally located onto the wound-remote side (distal position) of substrate (b).

When the layer (a) and substrate (b) have perforation holes, the second layer (c) also has preferably perforation holes. In that case, layer (a), substrate (b) as well as layer (c) preferably have the same pattern of perforation as far as location and size of the holes are concerned. In other words, the laminate comprising a second layer (c) is considered to be perforated by the perforation holes.

Further, the invention relates to an assembly comprising the laminate according to the invention and a textile or a polymer-based foil.

In line with this application a textile can be referred to as a flexible material which can be prepared by weaving, knitting, crocheting, knotting, tatting, felting, bonding or braiding of yarns or threads and thereby creating an interlocking network of these yarns or threads. These yarns or threads in turn can be obtained by spinning raw fibres from either natural or synthetic sources into long and twisted lengths. Examples of textiles are woven fabrics, non-woven fabrics, knittings, nettings or braids, tulle or felt.

In a preferred embodiment of the assembly of the invention, the textile is a woven fabric or a non-woven fabric.

A woven fabric is a fabric made by interlacing two or more threads at right angles to one another and can for example be obtained by weaving. Woven fabrics can be produced on a loom and made of many threads woven on a warp and a weft.

A non-woven fabric is a fabric-like material which can be made from staple fibre (short) and long fibres (continuous/long) bonded together by chemical, mechanical, heat or solvent treatment.

In a preferred embodiment of the invention the woven fabric or the non-woven fabric is based on natural or synthetic fibers.

Natural fibres can be referred to as fibres having a natural origin such as plants, animals or minerals. Natural fibres generally can be used without having undergone a chemical transformation reaction. Examples are silk, cellulose, cotton, hemp, linen and wool.

Synthetic fibres can be referred to as fibres which have been either chemically synthesized or natural fibres which have been chemically transformed or modified. Examples are viscose fibres or fibres made from polymers such as poly(meth)-acrylate, polyamide, polyimide, polyamidimide, polyurethane, polyester such as polyethylene terephthalate and polybutylene terephthalate, polyether, poly-acrylnitrile, polyalkylene such as polyethylene and polypropylene.

In another preferred embodiment, the polymer-based foil comprised by the assembly has a solubility in water of less than 10 mg/l, more preferably less than 1 mg/ml, in particular from 0,0001 to 1 mg/ml; determined with column elution according to EU-Guideline RL67-548-EWG, annex V, chapter A6. The polymer-based foil can, for example, comprise polymers such as polyurethane, polyether urethane, polyester urethane, polyester, polypropylene, polyethylene, polyamide, polyvinylchloride, polyorganosiloxane (silicone), polyacrylate, polymethacrylate or mixtures thereof, preferably polyurethane.

It is preferred that the polymer-based foil exhibits a water vapor permeability of at least 300 g/m²/24 hrs, in particular at least 1000 g/m²/24 hrs and especially at least from 2000 g/m²/24 hrs to 5000 g/m²/24 hrs, determined according to DIN EN 13726, upright.

In a preferred embodiment the assembly comprises a laminate according to the invention and a textile or a polymer-based foil, wherein the laminate is arranged such that the layer (a) of the laminate is the wound contact layer (proximal position). It is further preferred that the laminate comprises a second layer (c) comprising a medically acceptable adhesive. The textile or the polymer-based foil, preferably a polymer-film, is arranged on said second layer comprised by the present laminate.

In an alternatively preferred embodiment, the assembly comprises a laminate according to the invention, a textile or a polymer-based foil and a layer comprising absorbent material. An absorbent material can be referred to as a material capable of receiving and subsequently retaining a liquid such as water or exudate. Absorbent materials are known in the art. In a preferred embodiment the absorbent material is a superabsorbent material based on a copolymer of acrylic acid and acrylamide or a foam, preferably a foam based on polyurethane. The layer of the absorbent material such as a foam can preferably have a surface being smaller than the surface of the second layer comprised by the laminate. It is further preferred that the layer of absorbent material is arranged on the distal side of or adhered to the centre of the second layer (c) comprised by the laminate, such that four rims of the second layer (c) are formed which are not covered by the layer of absorbent material and wherein the opposing rims may have substantially the same area size. Alternatively, if the laminate has a shape different from a rectangular form there may be a rim having a different shape. For example, the rim can be circular or elliptical. Usually, the rim will have the shape of the laminate. Usually, the rim will be circumferential. In a preferred embodiment the textile or polymer-based foil can be adhered to the medically acceptable adhesive of said rims of the second layer (c) comprised by the laminate. Put differently, the layer (a) of the present laminate can be used as wound contact layer, while the second layer (c) comprised by the laminate enables the formation of an assembly optionally having further layers.

Moreover, the invention relates to a wound care product comprising the layer according to the invention, the adhesive laminate according to the invention and/or the assembly according to the invention. As far as the layer (a), laminate and/or the assembly are concerned, substantially the same applies as described above. Preferably the wound care product is a wound dressing.

Further, the invention is directed to the use of the layer according to the invention, the laminate according to the invention or the assembly according to the invention as (A) a wound contact layer of a wound dressing, preferably in an absorbent wound dressing, wherein the adhesive layer is preferably configured to comprise a wound contact area and a skin contact area and/or (B) a backing layer of a wound dressing, preferably as a backing layer of an island-like wound dressing which means that the backing stretches beyond the outer borders of the other layers of the dressing (top view) and that the whole backing or at least the margin are adhesive ensuring fixation to the skin on all sides.

The layer according to the invention may be used as backing in a wound dressing having a pad, preferably a pad comprising an absorbent material, wherein the pad is directly in contact with the wound. It is preferred that the pad, preferably the pad comprising an absorbent material, is of such a size that the four rims of the first layer (a) overlap the pad and thus allow adherence to a surface, preferably the skin, in a circumferential manner. It is further preferred that the laminate does not comprise a second layer (c) comprising a medically acceptable adhesive. In other words, the substrate (b) of the laminate can act as the backing layer of a wound dressing.

In a preferred embodiment the laminate according to the invention is used as a wound contact layer of an island-like wound dressing. It is more preferred that the layer comprised by the laminate according to the invention is used as a wound contact layer of a wound dressing. The properties of the layer ensure an excellent adherence to the surface of the skin and enable a safe and easy removal without negatively affecting the surface of the skin or wound to which it has been applied.

As far as the layer (a) comprised by the laminate is concerned, the same as above applies. The island-like wound dressing preferably comprises a laminate having further a second layer (c) comprising a medically acceptable adhesive on which a layer of absorbent material is adhered. With refence to the above, the surface of the layer comprising the absorbent material is preferably smaller than the surface of the second layer comprised by the laminate and is preferably arranged on or adhered to the distal side of the centre of the second layer comprised by the laminate, such that the four rims of the second layer are formed. These rims which are not covered by the layer of absorbent material preferably have substantially the same area size. In a preferred embodiment the textile or a polymer-based foil can be adhered to the medically acceptable adhesive of said rims of the second layer comprised by the laminate. In other words, the first layer comprised by the present laminate can be used as the wound contact layer, while the second layer comprised by the laminate enables the formation of an assembly optionally having further layers.

In an alternative embodiment the laminate according to the invention can be used as a backing layer of a wound dressing. An adhesive backing layer having different zones of peel strength may be advantageous when covering medical equipment which is connected to a patient. Examples of such medical equipment are cannulas, drain tubes, ports, external fixators etc. In such cases it may be desired that the backing provides strong adhesion to the skin but has a zone of low adhesion for providing access to the medical equipment - like for example removal of a drain tube without uncovering the wound.

In an alternative embodiment the assembly according to the invention can be used as a backing layer of a wound dressing, in particular a wound dressing having a pad, preferably a pad comprising an absorbent material, wherein the pad is directly in contact with the wound. It is preferred that the pad, preferably the pad comprising an absorbent material, is of such a size that the four rims of the first layer overlap the pad to adhere to a surface, preferably the skin. It is further preferred that the assembly comprises a laminate comprising layer (a), substrate (b) and second layer (c) as well as a textile or a polymer-based foil, wherein the textile or a polymer-based foil is arranged on or adhered to the distal side of the second layer (c) comprising a medically acceptable adhesive. In other words, the textile or the polymer-based foil of the assembly can act as a backing layer of a wound dressing.

According to a further embodiment the layer comprises one medically acceptable adhesive material on one surface, wherein the peel strength of the medically acceptable adhesive material in one area of the surface is smaller than the peel strength of the medically acceptable adhesive material in another area of the surface and wherein this other area of is the entire remaining area of said surface. Perforations however may be present.

According to another embodiment another embodiment the layer, the laminate or wound dressing according to the invention comprises at least one active ingredient. Preferably, the active ingredient is present at least in the area which has the lower peel strength. Alternatively, in case of a wound dressing the active ingredient may be present in the wound pad and the layer or laminate present in the dressing has perforations. The given active ingredient may be a substance that has antimicrobial properties or that promotes wound healing. An example of an antimicrobial substance is silver and an example of a substance which promotes wound healing is zinc. The advantage provided by this embodiment is that the active ingredient is transferred from or through the layer which adheres to the wound to the injured tissue.

According to a further aspect of the invention the irradiation with an electron beam may be provided as electron beam lithography. Hereby, the area of lower peel strength or the area of higher peel strength can be provided in the form of patterns adjusted to the desired purpose or use. The patterns created by electron beam lithography may for example include repetitive shapes of crosses, stars, triangles, squares, hexagons, circles, ellipses, waves or grids. The electron beam lithography may be used to create an area imitating the shape of certain body regions resulting for example in a wound dressing for application to the forehead or the eye having reduced peel force in the area to be applied over the eyebrow. Also, the electron beam lithography may be used to create a layer which has areas surrounding the perforations which areas have increased peel force to encounter the fact that the likelihood of detachment is higher in the region of a perforation.

Preferably, the layer or laminate to be produced by the method disclosed herein has an upper surface and a lower surface and the medically acceptable adhesive material is located on at least one surface wherein the area on which the energy dose is applied is not the entire surface on which the medically acceptable adhesive is located. More preferably, the area on which the energy dose is applied is surrounded by another area on the same surface wherein said another area is not irradiated. The layer of laminate produced in this way has an area in the center of a surface which has a lower peel strength than the other area in the periphery. The advantage of this approach is that the irradiated area in the center of the surface is highly suitable as atraumatic wound contact layer while the surrounding area on the same surface keeps its maximum peel force strength and allows excellent adherence to healthy skin. Layers and laminates derived or derivable by this method are part of the invention as are wound dressings comprising such layers or laminates. Such layers, laminates and wound dressings may have the additional features, properties or parameters as disclosed herein and may be adapted to the desired need and purpose.

It is worth mentioning that the irradiation according to the present invention is not intended to sterilize the treated layer or laminate. A separate sterilization step is therefore recommended whenever the resulting product is intended to be used on open wounds. Such sterilization step should not be based on a method involving irradiation as the adhesive properties of the product may be changed in an unforeseen way. A sterilization based on the application of ethylene oxide is an example of a suitable approach to achieve a sterile product. In this context within the methods according to the invention the irradiation itself may be defined as an irradiation which does not result in the sterilization of the adhesive, the layer, the laminate or the wound dressing.

According to a special aspect of the invention the layer of laminate has a lower and an upper surface wherein both surfaces are covered with a medically acceptable adhesive and wherein at least an area of the lower and at least an area of the upper surface are irradiated as described herein.

According to a further aspect of the invention the laminate comprises a substrate which is a grid or net wherein the openings within the grid or net allow passage of wound exudate. The substrate has a lower and an upper surface wherein both surfaces are covered with a silicone-based adhesive. Preferably the substrate is made of polyester, more preferably of PET. Due to the open grid structure of such laminate it is barely possible to cover or coat only one surface with a medically acceptable adhesive. Therefore, in the scope of the invention one of the two surfaces may be irradiated as described herein to reduce or even deplete the peel force strength of the respective surface. The resulting grid-shaped laminate can act as long-term wound contact layer having atraumatic adherence towards skin and wound and may be-while in place on the wound - covered by appropriate absorbing materials without sticking to these. The wound exudate passes through the grid and is retained in the absorbing materials. When the absorbing materials are saturated, they may be replaced while the grid- or net-shaped laminate stays on the wound offering protection during the change.

Further, the invention is directed to the use of the layer according to the invention, the laminate according to the present invention or the assembly according to the present invention in a process for manufacturing a wound care product, preferably a wound dressing. In other words, the layer according to the invention, the laminate according to the present invention or the assembly according to the present invention can be used in in a process for manufacturing a wound care product, preferably a wound dressing.

### Experimental Part:

### 1. Peel strength

### 1.1 Test samples were prepared as follows:

A4 sheets of a laminate having a layer (a) of silicone-based adhesive with a thickness of 150 µm, a substrate (b) of polyurethane-based membrane with a thickness of 25 µm and a layer (c) of an acrylic-based adhesive with a thickness of 30 µm were provided. The laminate was placed between two release liners, which consisted of siliconized paper on the acrylic adhesive and a film of LDPE on the opposing side of the laminate which was covered with silicone adhesive. These laminates were perforated by kiss-cutting to obtain perforation holes with a diameter of 2.4 mm such that the laminate had an open area of 15%. The release liner on the side with the acrylic-based adhesive was removed and replaced with a new liner.

The test samples were submitted to an e-beam treatment with a Rhodotron device from IBA with a 10 MeV e-beam to treat the layer with different doses (see below Table A), wherein the dosimetry was carried out with Alanine in line with international standards. Samples of 20 cm x 21 cm were cut out, wherein the number of samples per dose was 5. As control a laminate as described above which was not subjected to any irradiation was used.

**Table A**

| Treatment | Dose [kGy] |
|---|---|
| Control | 0 |
| 1 | 3.8 |
| 2 | 7.5 |
| 3 | 14.5 |
| 4 | 30.5 |
| 5 | 51.7 |

### 1.2 Determination of the peel strength

### 1.2.a Determination of the peel strength on Bristol paper

The peel strength of the samples on Bristol paper (Oxford, 180° angle) was determined by the method according to FINAT n°1 (FINAT Manuel Technique: Méthodes de Test. (2001). Niederlande: FINAT), wherein the peel strength was determined over a width of 25 mm five times per sample after dividing each sample into five specimens (cf. below Table B).

**Table B:**

| | Treatment Control | Treatment 1 | Treatment 2 | Treatment 3 | Treatment 4 | Treatment 5 |
|---|---|---|---|---|---|---|
| Peel strength sample 1 (N/in) | 1.97; | 1.85; | 1.72; | 1.21; | 0.31; | 0.04; |
| | 1.95; | 1.85; | 1.86; | 1.23; | 0.30; | 0.04; |
| | 1.91; | 1.82; | 1.79; | 1.19; | 0.30; | 0.03; |
| | 2.04; | 1.81; | 1.69; | 1.23; | 0.28; | 0.03; |
| | 2.05 | 1.84 | 1.64 | 1.20 | 0.28 | 0.03 |
| Peel strength sample 2 (N/in) | 2.01; | 1.96; | 1.76; | 1.29; | 0.23; | 0.04; |
| | 2.00; | 1.98; | 1.79; | 1.28; | 0.23; | 0.03; |
| | 2.05; | 1.94; | 1.80; | 1.15; | 0.24; | 0.03; |
| | 2.05; | 1.90; | 1.71; | 1.12; | 0.24; | 0.03; |
| | 1.95 | 1.93 | 1.76 | 1.10 | 0.30 | 0.03 |
| Peel strength sample 3 (N/in) | 1.96; | 1.91; | 1.70; | 1.13; | 0.29; | 0.03; |
| | 1.92; | 1.94; | 1.71; | 1.09; | 0.26; | 0.03; |
| | 1.97; | 2.02; | 1.75; | 1.14; | 0.23; | 0.04; |
| | 2.00; | 1.93; | 1.81; | 1.12; | 0.25; | 0.04; |
| | 1.98 | 1.93 | 1.67 | 1.11 | 0.25 | 0.04 |
| Peel strength sample 4 (N/in) | 2.00; | 1.99; | 1.81; | 1.14; | 0.29; | 0.04; |
| | 1.99; | 2.01; | 1.76; | 1.12; | 0.29; | 0.04; |
| | 2.00; | 2.00; | 1.76; | 1.15; | 0.25; | 0.04; |
| | 2.04; | 1.94; | 1.68; | 1.13; | 0.30; | 0.04; |
| | 2.06 | 1.85 | 1.66 | 1.12 | 0.27 | 0.04 |
| Peel strength sample 5 (N/in) | 1.94; | 1.83; | 1.80; | 1.23; | 0.30; | 0.03; |
| | 2.04; | 1.90; | 1.80; | 1.23; | 0.31; | 0.03; |
| | 1.97; | 1.84; | 1.80; | 1.30; | 0.30; | 0.03; |
| | 2.03; | 1.90; | 1.75; | 1.24; | 0.30; | 0.03; |
| | 1.99 | 1.83 | 1.75 | 1.17 | 0.27 | 0.03 |
| Average peel strength (N/in) | 1.99 | 1.91 | 1.75 | 1.18 | 0.27 | 0.03 |
| Standard deviation | 0.04 | 0.06 | 0.06 | 0.06 | 0.03 | 0 |

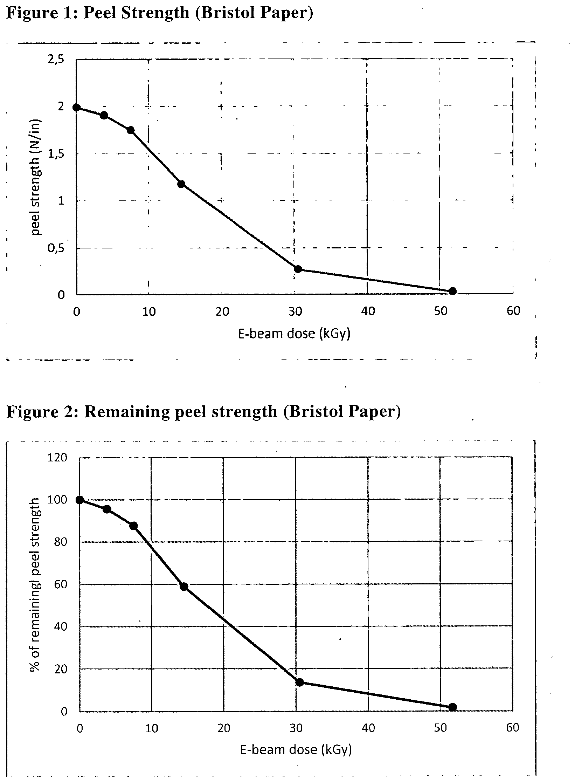

The results are shown in Figure 1. As can be seen from said Figure, as the dose obtained from the e-beam increases, the peel strength (and thus the adhesiveness) of the silicone-based adhesive decreases. Taking this into account, according to the present invention the adhesiveness of an area of a layer comprising a medically acceptable adhesive material can be adjusted depending on the dose applied. Thus, apart from consistently different levels of adhesive in areas of the layer it is also possible to achieve diffuse or variable levels from one area to the other such as for example from the center towards the borders (periphery) of a wound dressing.

Further, the remaining peel strength (in percentage of initial peel strength) of the samples which were irradiated by an energy dose was compared to the one of the control sample having 100% peel strength/adhesion (cf. below Table C)

**Table C**

| | Treatment Control | Treatment 1 | Treatment 2 | Treatment 3 | Treatment 4 | Treatment 5 |
|---|---|---|---|---|---|---|
| % of initial peel strength | 100 | 95.6 | 88.7 | 59.0 | 13.7 | 1.7 |

The results are shown in Figure 2. As can be seen from said Figure, the percentage of remaining peel strength decreases from 100% (control sample) with the energy dose applied to the sample.

### 1.2.b Determination of the peel strength on skin

The peel strength of the samples on skin (90° angle) was determined by the method according to FINAT FTM2 (Finat Technical Handbook TEST METHODS, Edition 2014 available via Laan van Nieuw-Oost Indië 131-G, 2593 BM The Hague, P.O. Box 85612, 2508 CH, The Hague, The Netherlands), but with the following adaptions: The specimens used had dimensions of 10 mm x 200 mm and were applied to the inner side of the forearm of a single test person. Subsequently the material was fixed by use of finger pressure (no use of a roller). Measurements have been taken 10 minutes after fixation (cf. below Table D).

**Table D:**

| | Treatment Control | Treatment 1 | Treatment 2 | Treatment 3 | Treatment 4 | Treatment 5 |
|---|---|---|---|---|---|---|
| Peel strength | 54.8; | 46.4; | 22.1; | 4.54; | 1.57; | 0; |
| | 55.2; | 45.3; | 28.8; | 3.92; | 1.47; | 0; |
| sample 1 (cN/cm | 61.1; | 43.6; | 26.8; | 5.71; | 1.73; | 0; |
| | 60.3; | 50.2; | 23.7; | 5.28; | 1.82; | 0; |
| | 64.8 | 45.4 | 21.3 | 5.28 | 2.12 | 0 |
| Peel strength sample 2 (cN/cm) | 46.7; | 39.5; | 22.9; | 4.42; | 1.99; | 0; |
| | 56.2; | 42.3; | 20.6; | 4.85; | 2.10; | 0; |
| | 57.2; | 40.8; | 19.5; | 5.28; | 1.69; | 0; |
| | 58.8; | 36.5; | 19.5; | 5.97; | 2.22; | 0; |
| | 57.6 | 41.0 | 18.8 | 6.07 | 1.72 | 0 |
| Average peel strength (cN/cm) | 57.3 | 43.1 | 22.4 | 5.13 | 1.84 | 0 |
| Standard deviation | 4.79 | 3.92 | 3.28 | 0.70 | 0.25 | 0 |

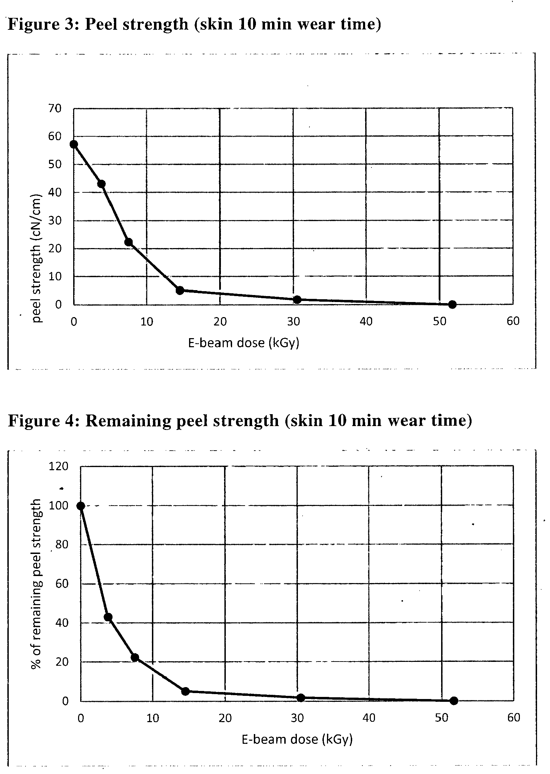

The results are shown in Figure 3. As can be seen from said Figure 3, similar as in Figure 1 as the dose obtained from the e-beam increases, the peel strength (and thus the adhesiveness) of the silicone-based adhesive decreases.

Further, the remaining peel strength (in percentage of initial peel strength) of the samples which were irradiated by an energy dose was compared to the one of the control sample corresponding to 100% peel strength/adhesion (cf. below Table E)

**Table E**

| | Treatment Control | Treatment 1 | Treatment 2 | Treatment 3 | Treatment 4 | Treatment 5 |
|---|---|---|---|---|---|---|
| % of initial peel strength | 100 | 43.1 | 22.47 | 5.1 | 1.8 | 0 |

The results are shown in Figure 4. As can be seen from said Figure, the percentage of the initial peel force (control sample at 100%) decreases with the energy dose applied to the sample.

### 2. Production of a Wound dressing

The same laminate as in the first experiment (a layer (a) of silicone-based adhesive, a substrate (b) of polyurethane film and a layer (c) of an acrylic-based adhesive) was provided in bulk on a roll as starting material for the production of a wound dressing on an assembling line. To make sure the laminate is processable without sticking to the assembling line, both sides of the laminate were covered by removable liners.

The liner on the silicone adhesive was a clear film made of low density polyethylene (LDPE) and the liner on the acrylic adhesive side was made of siliconized paper.

First, the laminate was mechanically unwound. Then it was perforated via kiss cutting (whole diameter of 2.4 mm, 15% open area). The liner of LDPE was removed from the silicone adhesive and the silicone adhesive was irradiated with an e-beam device by applying a dose of 25 kGy on an area intended as wound contact layer in the finalized product. A protective liner has been applied over the whole surface of the silicone adhesive. Subsequently, the release liner from the acrylic-based adhesive was removed and a wound pad (nonwoven comprising viscose) and a backing (PU film) are applied on said acrylic-based adhesive.

The preformed wound dressings were die-cut. Each dressing was sealed in a gas permeable package. Then the dressings were sterilized by application of ethylene oxide (EO). After removal of the EO, batches of five sterile-packed wound dressings were each fed into an outer carton, which then was sealed.

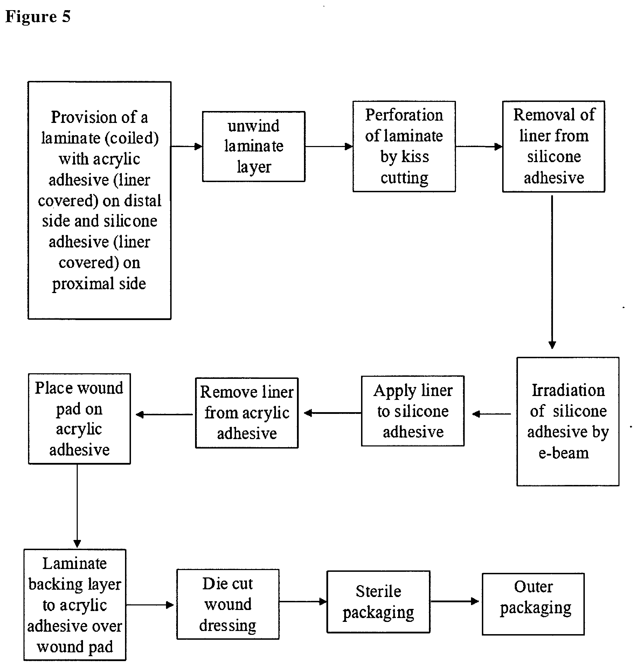

A diagram of the continuous process (work flow) is shown in Figure 5. By this process, wound dressings were obtained which have an advantageously high peel strength of 1,81 N / 25 mm in the periphery. While in use this location corresponds to the healthy skin surrounding the wound and thereby ensures that the wound dressing adheres well such that an undesired slipping or even removal of the wound dressing is prevented. In addition, the center of the dressing that covers the wound bed in use has a lower peel strength of 0.15 N / 25 mm, so that pain and / or damage to the wound is prevented when the dressing is removed or during a dressing change.

## Claims

1. Layer comprising one medically acceptable adhesive material on the same surface, wherein the peel strength of the medically acceptable adhesive material in one area of the layer is lower than the peel strength of the medically acceptable adhesive material in another area of the layer.

2. Layer according to claim 1, wherein the peel strength of the medically acceptable adhesive material in the center of the layer is lower than the peel strength in the periphery of the layer.

3. Layer according to claim 1 or 2, wherein the layer is a wound contact layer.

4. Layer according to any one of claims 1 to 3, wherein the medically acceptable adhesive material comprised by the layer is a silicon-based adhesive material.

5. Layer according to any one of claims 1 to 4 having perforation holes, wherein the perforation holes are evenly distributed over the whole area of the layer.

6. Layer according to any one of claims 1 to 5, wherein the openings of the perforation holes have a size of between 1.75 mm² and 12.5 mm².

7. Layer according to any one of claims 1 to 6, wherein the perforated surface is between 10% and 50% of the total surface of the layer.

8. Method for preparing a layer according to any one of claims 1 to 7 comprising the steps of
(I) providing a layer comprising one medically acceptable adhesive material
(II) applying an energy dose on at least one area of the medically acceptable adhesive material to reduce the peel strength of the medically acceptable adhesive material in this area by irradiating with an electron beam.

9. Method according to claim 8, wherein the energy dose of step (II) is within a range of 3 to 75 kGy.

10. Laminate comprising
(a) a layer according to any one of claims 1 to 7, and
(b) a substrate
wherein layer (a) is located on one side of the substrate (b).

11. Laminate according to claim 10, wherein the substrate is a film.

12. Laminate according to claim 10 or 11, wherein the laminate comprises a second layer (c) comprising a second medically acceptable adhesive material.

13. Laminate according to claim 12, wherein layer (a) is located on one side of the substrate (b) and wherein the second layer (c) is located on the opposite side of the substrate (b).

14. Assembly including the laminate according to any one of claims 10 to 13 and a textile or a polymer-based foil.

15. Use of the layer according to any one of claims 1 to 7, the laminate according to any one of claims 10 to 13 or the assembly according to claim 14 as
(A) wound contact layer of a wound dressing, preferably in an absorbent wound dressing, wherein the adhesive layer is preferably configured to comprise a wound contact area and a skin contact area
and/or
(B) a backing layer of a wound dressing, preferably as a backing layer of an island-like wound dressing.
